# EUROPEAN PATENT APPLICATION

(11) **EP 1 707 228 A1**
(43) Date of publication of application: **04.10.2006**
(21) Application number: 06250108.5
(22) Date of filing: 10.01.2006
(51) Int. Cl.: A61M 5/30

(54) **Patch with needleless injection systems**

(30) Priority: 28.03.2005 US 90883; 04.08.2005 US 196588
(71) Applicant: Van Laar, Kurt Daniel, California 93062-2232 (US)
(72) Inventor: Van Laar, Kurt Daniel, California 93062-2232 (US)
(74) Representative: Jennings, Nigel Robin

(57) **Abstract**

A needleless injection system comprises a patch body (90) for application to the skin of a person. The patch body includes a plurality of injectors (80), each of which comprises a cavity divided into two portions (15, 70) by a movable member (40; 200). One portion (15) contains a selectively actuable gas-generating compound (10) and the other portion (70) contains an injectable preparation and communicates with one surface of the patch body via an aperture (60), which is normally sealed by a rupturable membrane (50). The injection patch also includes a power source (135) connected to the gas-generating compound (10) in each cavity via a microprocessor (100), which is programmed to connect the power source (135) to the gas-generating compound in each cavity at predetermined times so as to cause the gas-generating compound to generate gas. The movable member is then caused to move within the associated cavity thereby pressurising the injectable preparation and causing the rupture membrane (50) to rupture and discharging the injectable preparation through the aperture (60).

## Description

The present invention relates to needleless systems for injecting medicinal or other preparations into the body of a patient. The preparation may be a pharmaceutical preparation or it may be a pain management or anaesthetic preparation or a control or incapacitating agent.

It is known to deliver pharmaceutical preparations into the body of a patient by means of so-called transdermal patches. Such patches are substantially planar composite sheets or patches of material including a support layer carrying a pharmaceutical preparation dissolved in an appropriate solvent that is capable of passing through the epidermis of a patient. The patch is adhered to the skin of the patient and the solvent progressively diffuses or permeates through the skin and into the bloodstream of the patient carrying with it the pharmaceutical preparation. Such patches may only be used with pharmaceutical preparations whose molecules are sufficiently small to be able to pass through the epidermis and this is a major limitation on the field of applicability of such patches. Furthermore, such patches do not permit any variation in the timing or rate of delivery of the pharmaceutical preparation. Thus the pharmaceutical preparation will start to be delivered shortly after application of the patch to the skin of the patient and the rate of delivery is determined solely by the size of the pharmaceutical molecules and the properties of the solvent. This is a further major limitation on the usage of such patches.

Needleless injection systems are also known which deliver a fine, high speed jet of a pharmaceutical preparation in a solvent against the skin of a patient. At least a proportion of the solvent and pharmaceutical preparation passes through the skin of the patient and thus into his bloodstream. Such systems are bulky and must be applied and manually actuated by a person on each occasion that an injection is required.

Needleless injection systems are also known in which the force needed to create the high speed jet of a pharmaceutical preparation in a solvent is produced by a pyrotechnic or gas-generating charge which is ignited or actuated at a selected time to deliver the pharmaceutical preparation transdermally. Examples of such known systems are disclosed in US-A-6352506 and US-A-6730028. In both cases, the skin must be ablated to permit the jet of pharmaceutical preparation to pass through a damaged or disrupted epidermis. US-A-4089334 discloses a needleless injection system with a mechanical actuation or firing mechanism incorporating a firing pin and cap mechanism to ignite the pyrotechnic or gas-generating charge. This system is bulky and does not permit the programmed delivery of a pharmaceutical preparation in selected amounts at selected time intervals.

It is the object of the invention to provide a needleless pharmaceutical delivery system that may be worn in the manner of a transdermal patch and that can sequentially deliver successive doses of an injectable preparation in predetermined amounts and at predetermined time intervals.

According to the present invention, a needleless injection system comprises a patch body for application to the skin of a person, the patch body including a plurality of injectors, each of which comprises a cavity divided into two portions by a movable member, the first portion of the cavity being substantially sealed and containing a selectively actuable gas generating compound and the second portion of the cavity containing an injectable preparation and communicating with one surface of the patch body via an aperture which is normally sealed by a rupturable member, the injection patch further including an initiating power source connected to the gas generating compound in each cavity via a microprocessor, the microprocessor being programmed to connect the initiating power source to the gas-generating compound in each cavity at predetermined times so as to cause the gas generating compound to generate gas, whereby the movable member is caused to move within the associated cavity thereby pressurising the injectable preparation and causing the rupture member to rupture and discharging the injectable preparation through the aperture.

Thus the injection system in accordance with the invention has an appearance and may be used in a manner similar to that of a conventional transdermal patch. However, it includes a plurality of injectors which are under the control of a microprocessor. Each injector comprises a cavity containing a movable member which divides it into two portions. One of these portions is substantially sealed and contains a gas-generating compound which may be selectively actuated under the control of the microprocessor. The other portion of the cavity contains an injectable preparation. The other portion of the cavity communicates with one surface of the patch, that is to say that surface which, in use, is situated against the skin of a patient, via an orifice, which is normally sealed by a rupturable membrane. When the gas-generating charge is actuated under the control of the microprocessor, a large volume of gas is produced virtually instantaneously which substantially increases the pressure in the first portion of the cavity. This pressure increase results in movement of the movable member which in turn produces an abrupt substantial increase in the pressure in that portion of the cavity containing the injectable preparation. This increase in pressure ruptures the rupturable membrane and the injectable preparation is then discharged through the aperture in the form of a rapid fine jet. This jet is sufficiently fine and fast that it penetrates the skin of the patient, whereby the preparation is injected into the patient. This injection may be relatively shallow and thus subcutaneous or relatively deep and thus intramuscular. The depth of penetration of the injection may be selected at will by varying the volume of the gas-generating charge, the diameter of the injection aperture and the viscosity of the injectable preparation.

In one embodiment of the invention, each injector includes a barrel, secured within which is a deformable membrane, e.g. of silicone rubber, which divides the cavity into two portions. The margin of the diaphragm will be fixed with respect to the barrel but an increase in pressure in the first portion of the cavity will result in deformation of the diaphragm, that is to say movement of a portion of it into the second portion of the cavity, which will result in the necessary pressure increase in the second portion of the cavity. This diaphragm might potentially be damaged by the heat produced when the gas-generating charge is activated and it is therefore preferred that a heat protective member in the form of a porous ceramic disc or the like is situated between the gas-generating compound and the diaphragm and thus protects the diaphragm from the heat produced.

In an alternative embodiment, each injector includes a barrel, movably located within which is a piston which divides the cavity into two portions. The injector is thus similar to a cylinder containing a movable piston. When the gas-generating charge is activated, the pressure increase in the first portion of the cavity results in movement of the piston which in turn produces a substantial increase in the pressure in the second portion of the cavity.

The gas-generating compound may take various forms and may for instance be sodium azide and/or a mixture of urazole with potassium perchlorate, optionally including a proportion of cordite in each case which will act as a controlled release gas generator.

The times at which the different injectors are actuated may be determined by the microprocessor and in this event the microprocessor will be programmable with the times at which at least some of the injectors are to be automatically actuated. However, it may be desirable for some or all of the injectors to be actuable in response to a command by the patient or a doctor. The patch may therefore include a sensor which is connected to the microprocessor and is responsive to a remotely transmitted signal to transmit a signal to the microprocessor to cause it to actuate a selected one or more of the injectors. Thus the patient or a doctor will carry a transmitter and if he should decide that an injection is required he will simply operate the transmitter to cause a selected one of the injectors to operate immediately in response to the signal produced by the transmitter, which is received by the sensor on the patch.

The injectable composition in the second portions of the cavities may be one or more of a pharmaceutical preparation, e.g. a preparation for diabetes management, a control or incapacitating agent and a pain management agent. It will be appreciated that it is not essential that all of the injectors contain the same injectable preparation and thus the cavities may contain two or even more different injectable preparations.

It is preferred that the patch is connected to the skin of a patient by an adhesive layer. This adhesive layer may be peelable but it is preferred that the adhesive is such that the adhesive connection may only be readily released with the aid of a solvent.

If the patient should inadvertently or deliberately remove the patch from his skin or otherwise tamper with it, it is desirable that the patch then be disabled so that no further actuation of any of the injectors occurs. It is therefore preferred that the patch includes a sensor which is responsive to removal of the patch from the skin of a patient or to other tampering with the patch and is arranged to transmit a tamper signal to the microprocessor which is programmed to disable the system on receipt of this tamper signal.

Further features and details of the invention will be apparent from the following description of two specific embodiments of needleless injection patch in accordance with the invention which is given by way of example only with reference to the accompanying drawings, in which:
Figure 1 is a diagrammatic plan view of the top or outer surface of a first embodiment;
Figure 2 is a diagrammatic and simplified sectional view on the line AA-AA in Figure 1;
Figure 3 is a diagrammatic sectional view on the line BB-BB in Figure 2;
Figure 4 is a view similar to Figure 3 of a second embodiment;
Figure 5 is a simplified diagrammatic view showing the electrical components of the patch;
Figure 6 is a diagrammatic plan view of the first or second embodiment showing the general layout;
Figure 7 is a sectional view on the line CC-CC in Figure 6; and
Figure 8 is a diagrammatic view similar to Figure 6 showing the electrical components of the first and second embodiments.

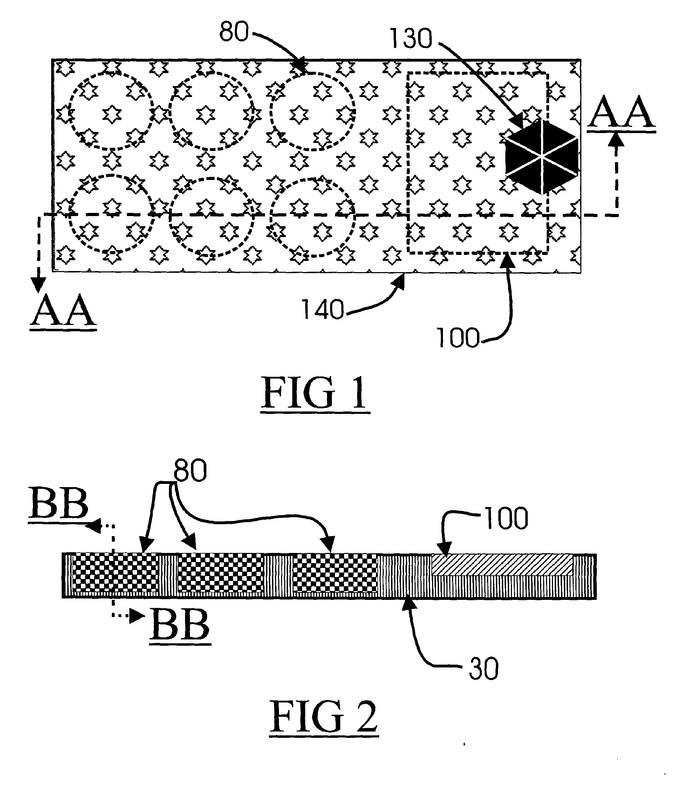

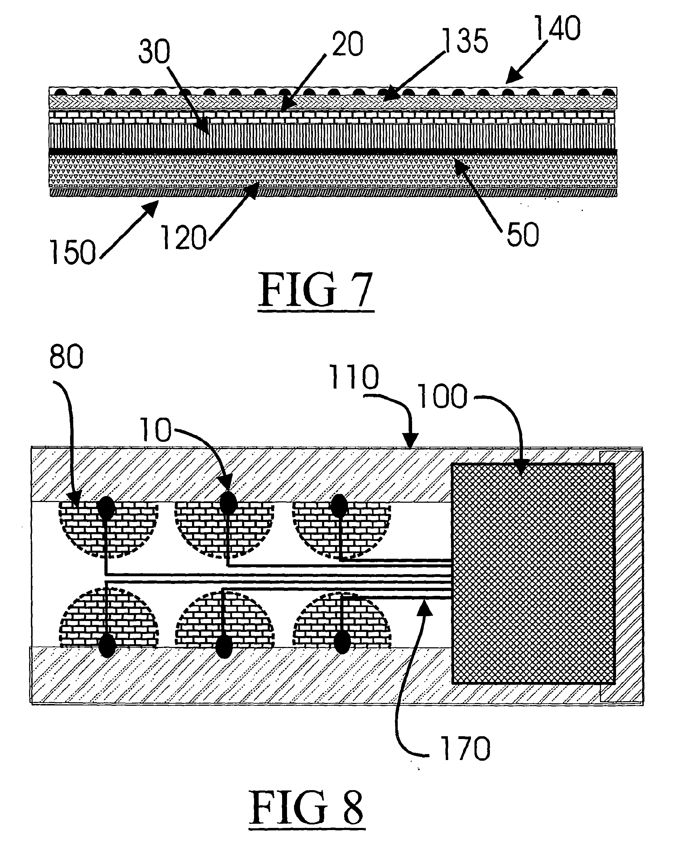

Referring firstly to Figures 1 to 3, the patch 90 is a generally laminar flexible composite strip of material whose appearance is generally similar to that of a conventional transdermal patch. It is typically 80 to 150 mm long, 12 to 40 mm wide and 10 to 15 mm thick. It comprises a central layer 30 of polymer material, e.g. of silicon rubber, embedded in which is a plurality of injectors 80, which will be described in more detail below. Secured to the top surface of the layer 30 is a gas-retaining layer 20, which in this case comprises a silicone adhesive reinforced with glass and silicon fibres. Secured to the top surface of the layer 20 is a battery in the form of a layer 135 constituting an electrical power source. Secured in turn to the top surface of the layer 135 is a sealing layer 140 of adhesive backed tape, made of release material, such as that sold under the Trade Mark TEFLON. Secured to the underside of the layer 30 is a continuous sealing membrane 50 of rupturable material. Secured to the underside of the membrane 50 is a die cut layer 120 of skin-compatible adhesive. Prior to use, the underside of the adhesive will in practice be covered by a peelable layer of release paper or the like, and such a layer is designated 150 in Figure 7.

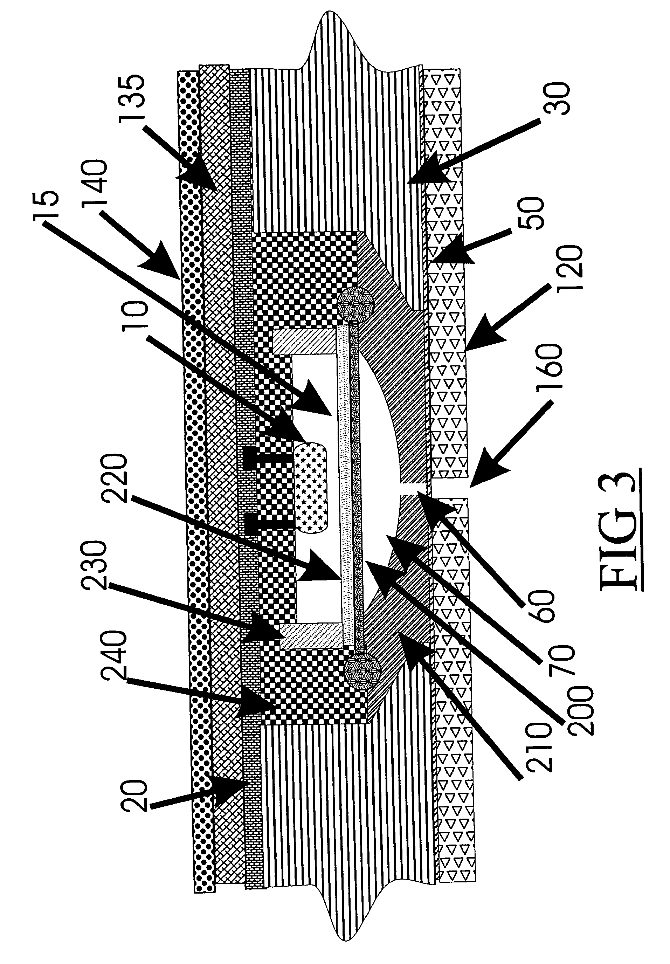

One of the injectors 80 is shown in more detail in Figure 3. It comprises a barrel defined by a top assembly 140, constituting the top and side wall of the barrel, and a bottom assembly 210, which constitutes the base of the barrel. Adjacent edges of the top and bottom assemblies 140, 120 are connected together with the interposition of the edge of a flexible diaphragm 200, which divides the interior of the barrel into two portions, an upper portion 68, which is sealed from the atmosphere, and a lower portion 70, which will accommodate the injectable pharmaceutical preparation. Formed in the bottom of the bottom assembly 210 is an injection orifice 60. Formed in the layer of adhesive 120 in alignment with the injection orifice 60 is an outlet orifice 160, which is slightly larger than the orifice 60. The orifice 60 is normally sealed by the membrane 50 which extends across it and the lower portion 70 of the injector cavity thus normally does not communicate with the atmosphere. Situated above the diaphragm 200 and retained in position by a porous ceramic spacer 230 is a porous heat-protective ceramic disc 220. Situated within the upper portion 68 of the cavity is a pyrotechnic or gas-generating charge 10. The charge 10 may be of various different types but is electrically triggerable, that is to say it must be capable of activation by an electric current or signal. Thus it may comprise one or more of sodium azide, optionally with an amount of cordite to act as a trigger or initiator, a mixture of urazole and potassium perchlorate in a stoichiometric ratio or other known gas-generating compounds. To enable ready actuation of the charge 10 it may include a so-called squib, which may be thought of as a trigger or detonator. This is a portion of the charge which may be readily ignited by a small electrical current and will then ignite the reminder of the charge. This may be achieved by providing a proportion of lamp black or conductive carbon in that region of the charge 10 adjacent the electrical connections. A similar effect can be achieved with nitrocellulose and acetone.

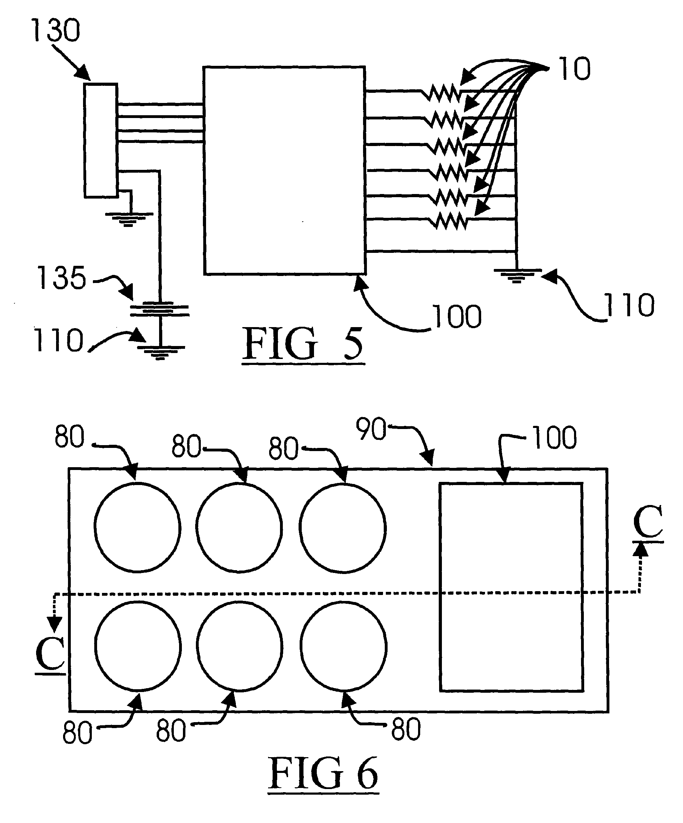

Also embedded in the patch is a programmable microprocessor 100. This is connected to the injectors 80 by respective electrical connections 170, which may be of screen printed type or of deposited copper type, as is used in printed circuit boards, or simply wires. The microprocessor is also connected to a control and programming connector 130, which may optionally include a signal receiver, and, via the connector 130, to the electrical power source 135, which in this case is a battery but may be a supercapacitor. The power source 135 and the gas generating charges are also connected to an electrical ground layer 110, as shown diagrammatically in Figure 5.

In use, the cavities 70 of the patch are filled with an injectable preparation. This may be of the same type and of the same amount in each cavity but the amounts and concentrations may vary from cavity to cavity and different cavities may contain quite different preparations. The programming connector 130 is connected to a programming device which programs the microprocessor to apply a predetermined electric current or signal to each electrical connector 170 at a respective predetermined time. The release paper 150 is removed and the patch then applied to the skin of the patient, e.g. on the upper arm, to which it is adhered by the adhesive 120. This may be of readily peelable type but it is preferred that it is of a type that is quasi-permanent and may only be removed with the aid of an appropriate solvent so as to prevent inadvertent or even deliberate removal by the patient. When the first predetermined time set in the microprocessor occurs, the microprocessor causes an electric current or signal to be passed from the power source 135 to the squib of the associated injector. This causes the squib to "fire" and thus ignite the associated charge 10 and rapidly produce a relatively large volume of gas. The porous ceramic disc 220 protects the diaphragm from the heat which is produced but the sudden increase in pressure in the cavity 68 results in the diaphragm 200 being deformed and pressurising the medicinal preparation in the cavity 70. This increase in pressure ruptures the membrane 50 sealing the orifice 60 and results in the medicinal preparation being expelled in a fine powerful jet through the orifice 160 against the skin of the patient, through which it passes. The preparation is thus supplied subcutaneously or intramuscularly, as required. The depth to which the preparation passes may be selectively controlled by appropriately varying the size of the gas generating charges, the diameter of the orifice 60 and the viscosity of the medicinal preparation. The remaining injectors are then triggered successively at the predetermined times programmed into the microprocessor.

Under certain circumstances it may be desirable for some or all of the injectors 80 to be under the manual control of the patient or a doctor or other attendant. In this event, the person who is to be able to control the injector(s) in question will carry a signal transmitting unit which may be operated to transmit a signal to the receiving unit connected to the microprocessor to command the microprocessor to trigger the selected injector(s) at any desired time.

The injection patch includes a sensor (not shown) which is connected to the microprocessor and is arranged to detect if the patch has been removed from the skin of the patient or otherwise tampered with and then to produce a tamper signal. The microprocessor is programmed to respond to this tamper signal by disabling or deactivating the patch so that no further injectors will be actuated until the patch has been reapplied and the microprocessor reset.

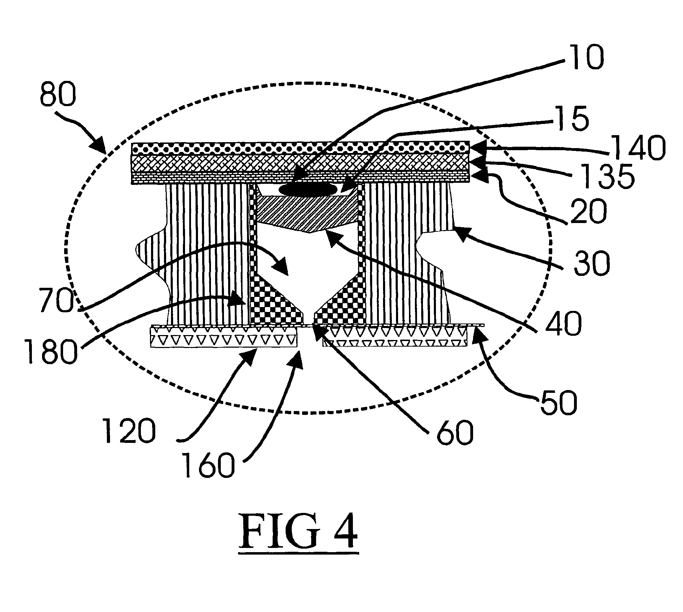

The modified embodiment shown in Figure 4 is very similar but the two-part barrel 210, 240 is replaced by a one-part barrel 180. This is open-topped but sealed at the top by the gas-retaining strip 20. The diaphragm 200 is replaced by a movable piston 20, which can slide within the barrel or cylinder 180. When the squib is fired, the increase in pressure in the cavity 15 results in movement of the piston 40, which pressurises the medicinal preparation in the cavity 70 which in turn again results in rupture of the membrane 50 and expulsion of the medicinal preparation through the orifice 60 in a powerful fine jet.

## Claims

1. A needleless medicinal injection system comprising a patch body for application to the skin of a person, the patch body including a plurality of injectors, each of which comprises a cavity divided into two portions by a movable member, the first portion of the cavity being substantially sealed and containing a selectively actuable gas generating compound and the second portion of the cavity containing an injectable preparation and communicating with one surface of the patch body via an aperture which is normally sealed by a rupturable member, the injection patch further including an initiating power source connected to the gas generating compound in each cavity via a microprocessor, the microprocessor being programmed to connect the initiating power source to the gas-generating compound in each cavity at predetermined times so as to cause the gas generating compound to generate gas, whereby the movable member is caused to move within the associated cavity thereby pressurising the injectable preparation and causing the rupture member to rupture and discharging the injectable preparation through the aperture.

2. A system as claimed in Claim 1 in which each injector includes a barrel, secured within which is a deformable diaphragm which divides the cavity into two portions.

3. A system as claimed in Claim 2 including a heat protective member in the form of a porous ceramic disc or the like situated between the gas-generating compound and the diaphragm.

4. A system as claimed in Claim 1 in which each injector includes a barrel, movably located within which is a piston which divides the cavity into two portions.

5. A system as claimed in any one of the preceding claims in which the gas-generating compound comprises one or more of sodium azide or a mixture of urazole with potassium perchlorate, optionally including a proportion of cordite.

6. A system as claimed in any one of the preceding claims in which the microprocessor may be programmed with the times at which at least some of the injectors are to be automatically actuated.

7. A system as claimed in any one of the preceding claims including a sensor which is connected to the microprocessor and is responsive to a remotely transmitted signal to transmit a signal to the microprocessor to cause it to actuate a selected one or more of the injectors.

8. A system as claimed in any one of the preceding claims in which the injectable composition in the second portions of the cavities is one or more of a pharmaceutical preparation, a control or incapacitating agent and a pain management agent.

9. A system as claimed in Claim 8 in which the second portion of some of the cavities contain an injectable preparation which is different to that in the second portion of others of the cavities.

10. A system as claimed in any one of the preceding claims in which the patch carries an adhesive layer adapted to connect the patch to the skin of a patient, the adhesive connection only being readily releasable with the aid of a solvent.
